# EUROPEAN PATENT SPECIFICATION

(11) **EP 1 335 677 B1**
(45) Date of publication and mention of the grant of the patent: **14.11.2007**
(21) Application number: 01937402.4
(22) Date of filing: 16.05.2001
(51) Int. Cl.: A61B 18/14

(54) **SYSTEM OF TREATING ABNORMAL TISSUE IN THE HUMAN ESOPHAGUS**
SYSTEM ZUM BEHANDELN VON ABNORMALEM GEWEBE IN DER MENSCHLICHEN SPEISERÖHRE
SYSTEME DE TRAITEMENT DE TISSUS ANORMAUX DE L'OESOPHAGE DE L'HOMME

(30) Priority: 16.11.2000 WO PCT/US00/31561
(43) Date of publication of application: 20.08.2003
(73) Proprietor: BARRX Medical, Inc., Sunnyvale CA 94089 (US)
(72) Inventor: Ganz, Robert, A., Minnetonka, MN 55305 (US); Stern, Roger A., Cupertino, CA 95014 (US); Zelickson, Brian D., Minneapolis, MN 55416 (US)
(74) Representative: Fennell, Gareth Charles
(86) International application number: PCT/US2001/015680
(87) International publication number: WO 2002/039915

(56) References cited:
- WO-A-00/01313
- WO-A-00/59393
- WO-A-00/66020
- WO-A-01/35846
- WO-A-99/35987
- WO-A-99/55245
- US-A- 5 833 688
- US-A- 6 071 277

## Description

### Field of the Invention

This invention relates to a system for treating abnormal epithelium in an esophagus.

### Background of the Invention

Two of the major functions of the human esophagus are the transport of food from intake to the stomach and the prevention of retrograde flow of gastrointestinal contents. The retrograde flow is largely prevented by a lower esophageal sphincter which normally remains closed and which functionally separates the stomach from the esophagus. In particular, the lower esophageal sphincter remains closed until neural activation causes its relaxation, allowing food to pass into the stomach from the esophagus. Certain conditions including hiatal hernia and loss of sphincter tone can cause inappropriate relaxation or malfunction of the sphincter. Various types of food and other activity can also cause relaxation of the sphincter, such as fatty meals, smoking and beverages having xanthine content. Some drugs or pharmaceuticals also may cause relaxation of this lower esophageal sphincter, as well as localized trauma or other problems such as neuromuscular disorders.

Those with persistent problems or inappropriate relaxation of the lower esophageal sphincter can develop a condition known as gastroesophageal reflux disease, manifested by classic symptoms of heartburn and regurgitation of gastric and intestinal content. As suggested above, the causative agent for such problems may vary. Patients with severe forms of gastroesophageal reflux disease, no matter what the cause, can sometimes develop secondary damage of the esophagus due to the interaction of gastric or intestinal contents with esophageal cells not designed to experience such interaction.

The treatment of gastroesophageal reflux disease, caused by malfunction of the lower esophageal sphincter, is not the subject of this patent application, rather the invention is focused on treatment of the secondary damage to esophageal tissue particularly a condition known as Barrett's esophagus.

The esophagus is composed of 3 tissue layers; an inner mucosa layer lined by squamous epithelial cells, a middle submucosa layer and an external muscle layer. When gastroesophageal reflux occurs, the inner squamous epithelial cells are exposed to gastric acid, along with intestinal bile acids and enzymes. This exposure may be tolerated, but in some cases can lead to damage and alteration of the squamous cells, causing them to change into taller, specialized columnar epithelial cells. This metaplastic change of the mucosal epithelium from squamous cells to columnar cells is called Barrett's esophagus, named after the British surgeon who originally described the condition.

Barrett's esophagus has important clinical consequences, since the Barrett's columnar cells can, in some patients, become dyplastic and then progress to a certain type of deadly cancer of the esophagus. The presence of Barrett's esophagus is the main risk factor for the development of adenocarcinoma of the esophagus.

Accordingly, attention has been focused on identifying and removing this abnormal Barrett's columnar epithelium in order to mitigate more severe implications for the patient. Examples of efforts to properly identify Barrett's epithelium or more generally Barrett's esophagus, have included conventional visualization techniques known to practitioners in the field. Although certain techniques have been developed to characterize and distinguish such epithelium cells, such as disclosed in United States Patent No. 5,524,622 and United States Patent No. 5,888,743, there has yet to be shown safe and efficacious means of accurately removing undesired growths of this nature from portions of the esophagus to mitigate risk of malignant transformation.

Devices and methods for treating abnormal body tissue by application of various forms of energy to such tissue have been described, and include laser treatment, radiofrequency ablation, ultrasonic ablation, photodynamic therapy using photo-sensitizing drugs, argon plasma coagulation, cryotherapy, and x-ray. These methods and devices are all defective however, since they do not allow for control of the depth of penetration of the energy means. This is a problem since uncontrolled energy application can penetrate too deeply into the esophageal wall, beyond the mucosa and submucosal layers, into the muscularis externa, potentially causing esophageal perforation, stricture or bleeding. In addition, most of these methods and devices treat only a small portion of the abnormal epithelium, making treatment of Barrett's time-consuming, tedious, and costly.

For example, International Patent Application Number PCT/US/00/08612 describes a method and apparatus for treating body structures involving unwanted features or other disorders. In one embodiment of that invention, a treatment device and method is described for treating a portion of the mucosal surface of the esophagus using the application of energy or other means. The device and method for treating the esophageal describes treating a limited arc of the esophageal tissue at a time and does not provide application of energy to effect ablation of tissue to a controlled depth.

WO 99/35987 describes an apparatus to treat a sphincter. The apparatus includes a support steerable member, having at its distal end two expandable members either contiguous or superimposed. One or both of the expandable members have a plurality of energy delivering means. One or both of the expandable members are porous or have perforations to allow the expanding medium, in the form of an electrolyte, to controllably wet the surface of the member and enhance energy transfer.

WO 01/35846 describes an ablation catheter system and method of use. The system and method are provided to endoscopically access portions of the human esophagus experiencing undesired growth of columnar epithelium. The ablation catheter system and method includes controlled depth of ablation features and use of either radio frequency spectrum, non-ionizing ultraviolet radiation, warm fluid or microwave radiation, which may also be accompanied by improved sensitizer agents.

Thus, a need exists for a device and method for treating abnormal tissue of the esophagus to a safely controlled shallow depth of ablation, restricting treatment to the mucosa and submucosa only, avoiding the muscle layer, through uniform application of energy to the tissue to be ablated, throughout the circumference of the inner lumen of the esophagus.

### Summary of the Invention

Aspects of the invention are defined in the accompanying claims.

An ablation catheter system is provided to endoscopically access portions of the human esophagus experiencing undesired growth of columnar epithelium. The ablation catheter system and method can include controlled depth of ablation features and use of either radio frequency spectrum, non-ionizing ultraviolet radiation, ultrasound, warm fluid or microwave radiation, collimated or non-collimated light or coherent or incoherent light energy, or other light energy, which may also be accompanied by improved sensitizer agents. An embodiment of the invention can provide for the uniform application of energy throughout the circumference of the inner lumen of the esophagus for ablation of cells in the mucosal and top portion of the submucosal layers of the esophagus while avoiding penetration of the muscle layer, also referred to as the muscularis externa.

### Brief Description of the Drawings

Figure 1 is a schematic view of portions of an upper digestive tract in a human.
Figure 2 is a schematic view of a device of the invention, in an expanded mode, within an esophagus.
Figure 3 is a schematic view of a device of the invention.
Figure 4 is a photograph of the device of Figure 3.
Figure 5 is a view of a device of the invention.
Figure 6 shows the electrode patterns of the device of Figure 3.
Figure 7 shows electrode patterns that may be used with a device of the invention.
Figure 8 is a schematic view of another embodiment of a device of the invention.
Figure 9 shows a top view and a bottom view of an electrode pattern of the device of Figure 8.

### Detailed Description of the Invention

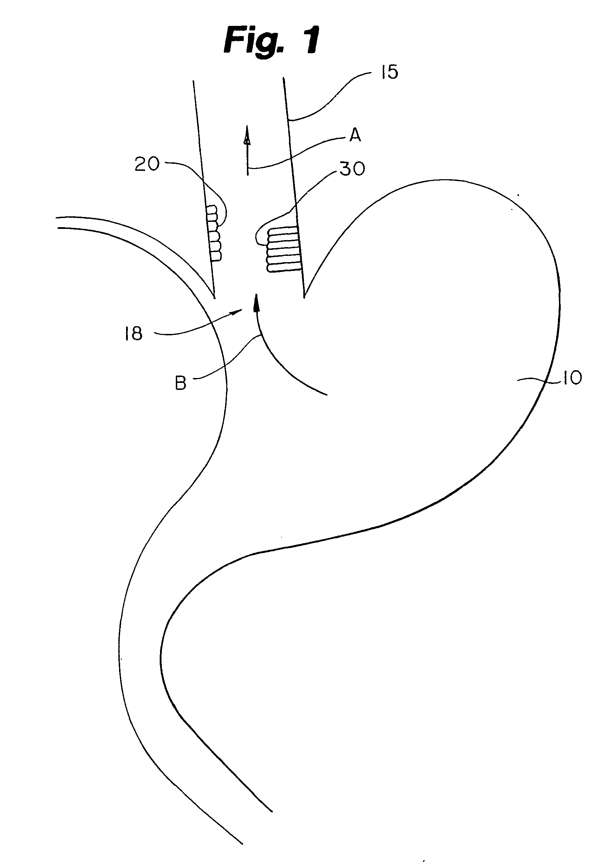

It is known that certain disorders can cause the retrograde flow of gastric or intestinal contents from the stomach 10 into the esophagus 15, as shown in Figure 1. This flow is shown by arrows A and B in Figure 1. Although the causation of these problems are varied, this retrograde flow may result in secondary disorders, such as Barrett's esophagus, which require treatment independent of and quite different from treatments appropriate for the primary disorder-such as disorders of the lower esophageal sphincter 18. Barrett's esophagus is an inflammatory disorder in which the stomach acids, bile acids and enzymes regurgitated from the stomach and duodenum enter into the lower esophagus causing damage to the esophageal mucosa. Indeed, when this type of retrograde flow occurs frequently enough, damage may occur to esophageal epithelial cells 20. In some cases the damage may lead to the alteration of the squamous cells, causing them to change into taller specialized columnar epithelial cells. This metaplastic change of the mucosal epithelium from squamous cells to columnar cells is called Barrett's esophagus. It is well established that although some of the columnar cells may be benign, others may result in adenocarcinoma.

Accordingly, attention has been focused on identifying and removing this columnar epithelium in order to mitigate more severe implications for the patient. Examples of efforts to properly identify these growths, referred to as Barrett's epithelium or more generally as Barrett's esophagus, have included conventional visualization techniques known to practitioners in the field. Although certain techniques have been developed to characterize and distinguish such epithelium cells there has yet to be shown safe and efficacious means of accurately removing undesired growths and abnormal tissue of this nature from portions of the esophagus to mitigate risk of malignant transformation.

Means for achieving such removal growths and tissue according to this invention includes use of energy, such as radiofrequency energy, at appropriate levels to accomplish ablation of mucosal or submucosal level tissue. Such ablation is designed to remove the columnar growths 30 from the portions of the esophagus 15 so affected. The term "ablation" as used herein means thermal damage to the tissue causing tissue necrosis.

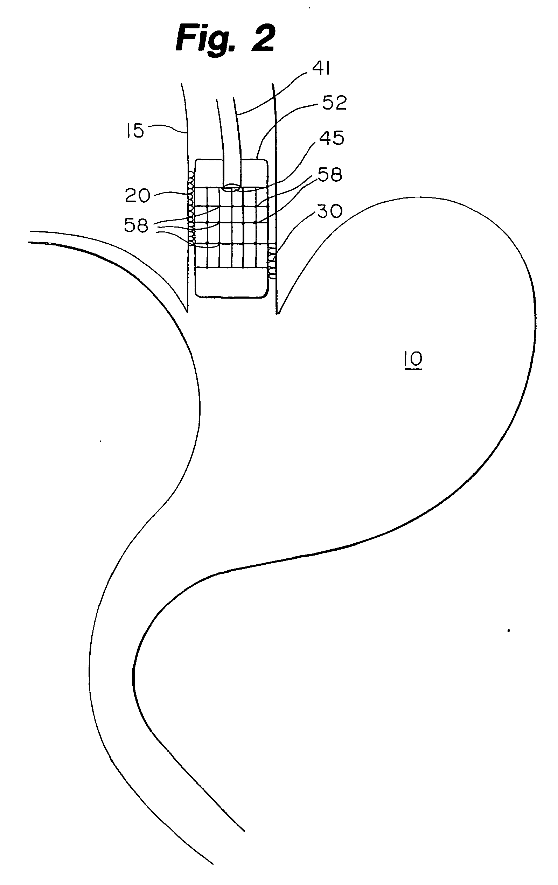

In one embodiment, as shown in Figure 2, an elongated flexible shaft 41 is provided for insertion into the body in any of various ways selected by the medical provider. The shaft may be preferably placed endoscopically, e.g. through the esophagus, or it may be placed surgically, or by other means. When an endoscope is used the flexible shaft may be inserted in the lumen of the endoscope or the shaft may be positioned on the outside of the endoscope. Alternately, an endoscope may be used to visualize the pathway the shaft should follow during placement and then following the removal of the endoscope the shaft could be inserted into the esophagus. Energy distribution means is provided at a distal end 45 of the flexible shaft to provide appropriate energy for ablation as desired. The energy distribution means are powered by power means configured for powering the energy distribution means at levels appropriate to ablate tissue to a predetermined depth of ablation. It is recognized that energy of a preferred type includes radio frequency energy, microwave energy, ultrasonic energy, or light energy, including without limitation, ultraviolet, visible, infrared, collimated or non-collimated, coherent or incoherent, or other light energy, the light energy possibly used in combination with improved sensitizing agents. It is also recognized that another embodiment of this invention may utilize heatable fluid as the ablation energy medium.

In one embodiment, the flexible shaft comprises a cable containing a plurality of electrical conductors surrounded by an electrical insulation layer and comprises a radiant energy distribution means located at its distal end. In one form of the invention, a positioning and distending device around the distal end of the instrument is of sufficient size to contact and expand the walls of the body cavity in which it is placed (e.g. the esophagus) both in the front of the distribution means as well as on the sides of the distribution means. For example, the distal head of the instrument can be supported at a controlled distance from, or in direct contact with the wall of the esophagus by an expandable balloon member 52 so as to regulate and control the amount of energy transferred to the tissue comprising the esophageal wall. The balloon is preferably bonded to a portion of the flexible shaft at a point spaced from the distal head means.
The distending or expandable balloon member can be used as the vehicle to deliver the ablation energy. A critical feature of this embodiment includes means by which the energy is transferred from the distal head portion of the invention to the membrane comprising the balloon member. For example, one type of energy distribution that may be appropriate and is incorporated herein in its entirety is shown in United States Patent No. 5,713,942, in which an expandable balloon is connected to a power source, which provides radio frequency power having the desired characteristics to selectively heat the target tissue to a desired temperature. The balloon 52 of the current invention may be constructed of an electroconductive elastomer such as a mixture of polymer, elastomer, and electroconductive particles, or it may comprise a nonextensable bladder having a shape and a size in its fully expanded form, which will extend in an appropriate way to the tissue to be contacted. In another embodiment, an electroconductive member may be formed from an electrically insulating elastomer wherein an electroconductive material such as copper is deposited onto a surface and an electrode pattern is etched into the material and then the electroconductive member is attached to the outer surface of the balloon member. In one embodiment, the electroconductive member, e.g. the balloon member 52, has a configuration expandable in the shape to conform to the dimensions of the expanded (not collapsed) inner lumen of the human lower esophageal tract. In addition, such electroconductive member may consist of a plurality of electrode area segments 58 having thermistor means or the like associated with each electrode segment by which the temperature from each of a plurality of segments is monitored and controlled by feedback arrangement. In another embodiment, it is possible that the electroconductive member may have means for permitting transmission of microwave energy to the ablation site. In yet another embodiment, the distending or expandable balloon member may have means for carrying or transmitting a heatable fluid within one or more portions of the member so that the thermal energy of the heatable fluid may be used as the ablation energy source.

One device of the invention, such as that shown in Figure 2, includes steerable and directional control means, a probe sensor for accurately sensing depth of ablation, and appropriate alternate embodiments so that in the event of a desire not to place the electroconductive elements within the membrane forming the expandable balloon member it is still possible to utilize the balloon member for placement and location control while maintaining the energy discharge means at a location within the volume of the expanded balloon member, such as at a distal energy distribution head of conventional design.

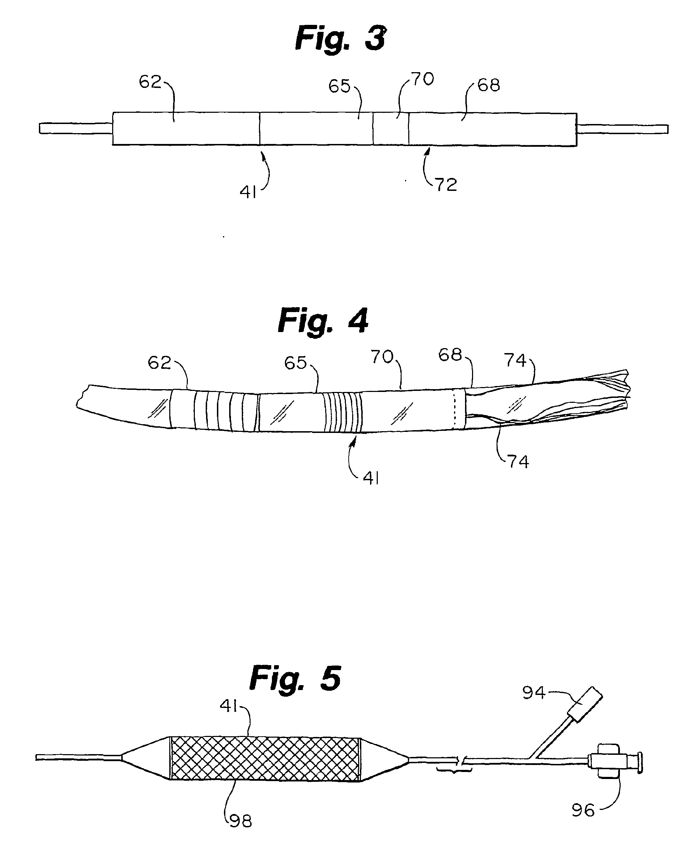

As shown in Figures 4, 5, and 8, in a preferred embodiment, the energy discharge or distribution means is positionable so that energy is uniformly applied to the circumference of the inner lumen of the esophagus where ablation is desired. In this embodiment, this is accomplished by positioning the energy discharge means on the outside circumference of the expandable balloon. One of ordinary skill would be able to position the energy discharge means used with the other energy sources with respect to the expandable member so that the energy is uniformly applied to the circumference of the inner lumen of the esophagus. One way to ensure that the energy is uniformly applied to the circumference of the inner lumen of the esophagus, would be include with the device a vacuum or suction element to "pull" the esophageal wall against the outside circumference of the expandable member. This suction element may be used alone to "pull" the esophageal wall into contact with the energy distribution means carried on the catheter or flexible shaft without the use of an expandable member or in conjunction with the expandable member to ensure the wall is in contact with the energy distribution means carried on the outside of the expandable member.

As described below, the power source may be manually controlled by the user and is adapted to allow the user to select the appropriate treatment time and power setting to obtain a controlled depth of ablation or it may be connected to a controller (not shown), which may be a digital or analog controller for use with the RF source, or a computer with software. When the computer controller is used it can include a CPU coupled through a system bus. The system may include a keyboard, a disk drive, or other non-volatile memory system, a display and other peripherals known in the art. A program memory and a data memory will also be coupled to the bus.

The depth of ablation obtained with the system of the invention is preferably controlled by the selection of appropriate treatment parameters by the user as described in the examples set forth herein. A probe sensor may also be used with the system of the invention to monitor and determine the depth of ablation.

The system disclosed herein may be utilized as a procedural method of treating Barrett's esophagus. This can include the detection and diagnosis of undesired columnar epithelium within the esophagus. After determining that the portion or portions of the esophagus having this undesired tissue should be partially ablated, then the patient is prepared as appropriate according to the embodiment of the device to be utilized. Then, the practitioner prepares the patient as appropriate and inserts, via endoscopic access and control, the ablation device shown and discussed herein through the mouth of the patient. As discussed above, the ablation device may be inserted through a channel of the endoscope, located on the outside of and along the side of the endoscope, or may be inserted through the mouth of the patient to the desired location in the esophagus without an endoscope after an endoscope has been used to identify the proper location and identify the path for insertion of the device. The device can be inserted with the endoscope and once the device has been inserted, further positioning of portions of the device will occur until proper location and visualization identifies the ablation site in the esophagus. Selection and activation of the entire circumferential electrode array or the appropriate quadrant(s) or portion(s)/segment(s) on the ablation catheter member is performed by the physician, including appropriate power and time settings according to the depth of ablation desired. Additional settings may be necessary as further ablation is required at different locations and/or at different depths within the patient's esophagus. Following the ablation, appropriate follow-up procedures as are known in the field are accomplished with the patient during and after removal of the device from the esophagus.

Following the ablation treatment to remove the Barrett's epithelium, the patient can be treated with acid suppression therapy, which has been shown to enhance the growth of normal epithelium during the healing process.

The ablation treatment with light energy may also be accompanied by improved sensitizer agents, such as hematoporphyrin derivatives such as Photofrin^{®} (porfimer sodium, registered trademark of Johnson & Johnson Corporation, New Brunswick, NJ).

In yet another embodiment of the method of the invention, the system disclosed herein may be utilized as a procedural method of treating dysplasia or cancerous tissue in the esophagus. After determining that the portion or portions of the esophagus having undesired tissue which should be partially or fully ablated, then the patient is prepared as appropriate according to the embodiment of the device to be utilized and treatment is provided as described above.

In yet another method of the invention, the practitioner may first determine the length of the portion of the esophagus requiring ablation by visual observation through an endoscope and then may choose an ablation catheter from a plurality of ablation catheters of the invention, each catheter having a different length of the energy discharge means associated with the expandable member. For example, if the practitioner determined that 1 centimeter of length of the esophageal surface required ablation, an ablation catheter having 1 centimeter of length of an electrode member or other energy distribution means that would apply energy to a length of 1 centimeter could be chosen for use in the ablation. The length of the electrode member or other energy distribution means associated with the balloon member can vary in length from 1 to 10 cm.

In yet another embodiment, a plurality of ablation catheters wherein the energy distribution means are associated with a balloon member can be provided wherein the diameter of the balloon member when expanded varies from 12mm to 35 mm. In this method, the practitioner will choose an ablation catheter having a diameter that when expanded which will cause the esophagus to stretch and the mucosal layer to thin out, thus, reducing blood flow at the site of the ablation. The esophagus normally is 5 to 6 mm thick, with the method of the invention the esophagus is stretched and thinned so that the blood flow through the esophageal vasculature is occluded. It is believed that by reducing the blood flow in the area of ablation, the heat generated by the radiant energy is less easily dispersed to other areas of the esophagus thus focusing the energy to the ablation site.

One means a practitioner may use to determine the appropriate diameter ablation catheter to use with a particular patient would be to use in a first step a highly compliant balloon connected to pressure sensing means. The balloon would be inserted into the esophagus and positioned at the desired site of the ablation and inflated until an appropriate pressure reading was obtained. The diameter of the inflated balloon would be determined and an ablation device of the invention having a balloon member capable of expanding to that diameter would be chosen for use in the treatment. It is well known that the esophagus may be expanded to a pressure of 60-120 Ibs./square inch. It is
desirable to expand the expandable electroconductive member such as a balloon sufficiently to occlude the vasculature of the submucosa, including the arterial, capillary or venular vessels. The pressure to be exerted to do so should therefore be greater than the pressure exerted by such vessels. Alternately, the practitioner may determine the appropriate diameter of ablation catheter to use with visual observation using an endoscope.

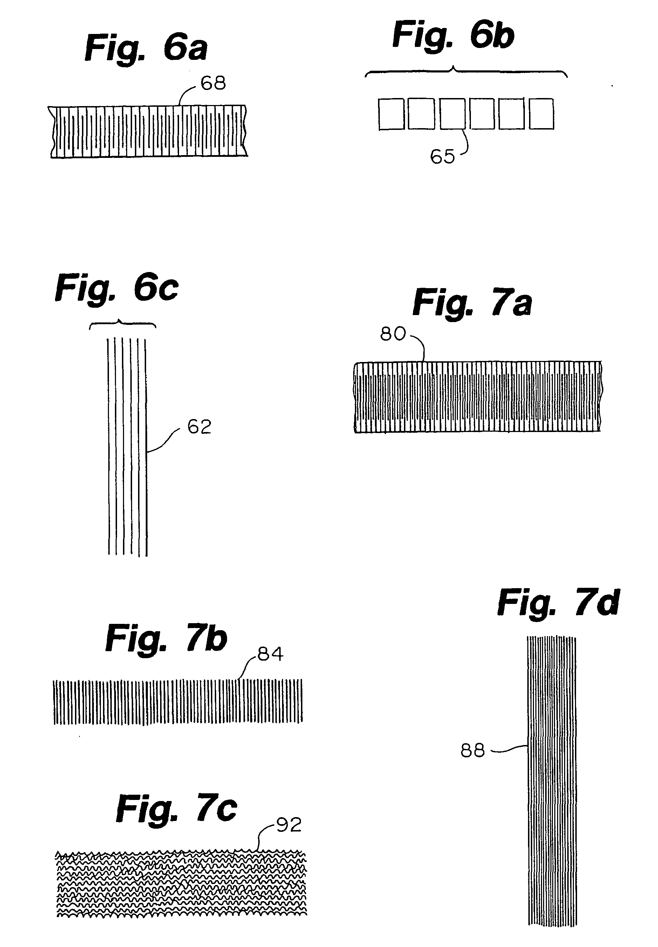

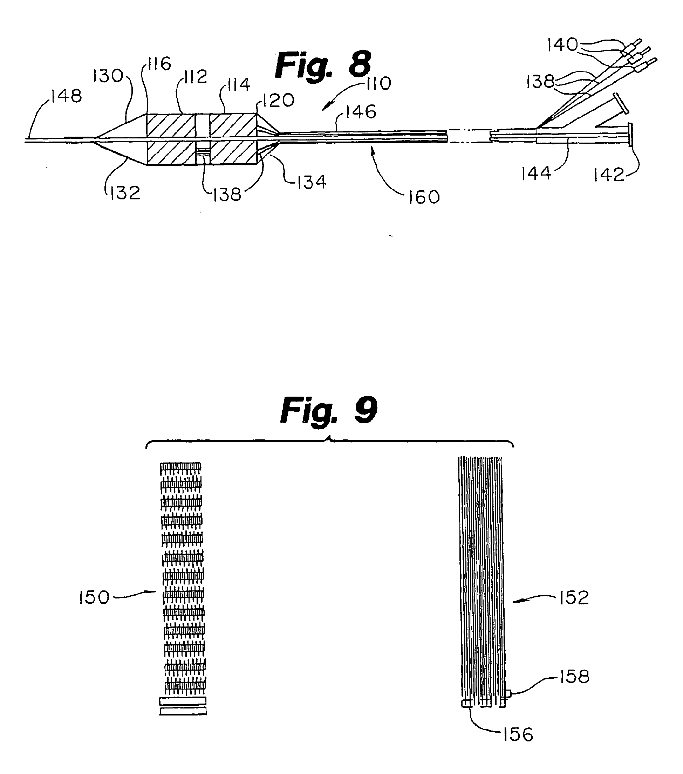

Operation and use of a device of the invention are described as follows. The device used is shown schematically in Figures 3, 4, and 5. As shown in Figure 5, the elongated flexible shaft 41 is connected to a multi-pin electrical connector 94, which is connected to the power source and includes a male luer connector 96 for attachment to a fluid source useful in expanding the expandable member. The elongated flexible shaft has an electrode 98 wrapped around the circumference. The expandable member of the device shown in Figures 3 and 4, a balloon, further includes three different electrode patterns, the patterns of which are represented in greater detail in Figure 6. Normally, only one electrode pattern would be used in a device of this invention, such as in the preferred device shown in Figure 8 and described below. In the device shown in Figures 3 and 4, the elongated flexible shaft 41 comprises six bipolar rings 62 with 2mm separation at one end of the shaft (one electrode pattern), adjacent to the bipolar rings is a section of six monopolar bands or rectangles 65 with 1mm separation (a second electrode pattern), and another pattern of bipolar axial interlaced finger electrodes 68 is positioned at the other end of the shaft (a third electrode pattern). In this device, a null space 70 was positioned between the last of the monopolar bands and the bipolar axial electrodes. The catheter used in the study was prepared using a polyimide flat sheet of about 1 mil (0.001") thickness coated with copper. The desired electrode patterns were then etched into the copper. This device is adapted for use with radiofrequency energy.

The electrode patterns of the invention may be varied depending on the length of the site to be ablated, the depth of the mucosa and submucosa at the site of ablation and other factors. Other possible electrode patterns which may be used with a device of the invention include, without limitation, those patterns shown in Figure 7 as 80, 84, 88, and 92, respectively. Pattern 80 is a pattern of bipolar axial interlaced finger electrodes with .3mm separation. Pattern 84 includes monopolar bands with 3mm separation. Pattern 88 includes bipolar rings with .3mm separation. Pattern 92 is electrodes in a pattern of undulating electrodes with .2548mm separation.

In the examples of use of the device described herein, the electrodes were attached to the outside surface of an esophageal dilation balloon 72 having a diameter of 18 mm. The device was adapted to use radio frequency by attaching wires 74 as shown in Figure 4 to the electrodes to connect them to the power source.

The balloon was deflated and the catheter inserted into the esophagus as described below. In addition to the series of three different electrode patterns, a number of different energy factors were applied to the esophagus of a normal immature swine (about 25 kgs). First, an endoscope was passed into the stomach of the subject. The device of the invention was placed into the distal esophagus using endoscopic guidance. The balloon member was inflated to press the electrodes against the esophageal mucosa. There was no indication that balloon dilation resulted in untoward effects on the esophagus.

Once the balloon member and electrodes were in place the first set of radio frequency ("RF") applications were made. Following endoscopic evaluation of the treated areas, the device was withdrawn proximally. The placement of the device was evaluated endoscopically to assure a gap of normal tissue between the area of the first application and the second application, which gap will assure identification of the two treatment areas during post procedure evaluations. The procedure was repeated a third time using a similar procedure to that of the second application. During the treatment the tissue impedance was monitored as an indicator of the progress of the treatment, high impedance being an indication of desiccation. Accordingly, the practitioner can determine through monitoring the tissue impedance when sufficient ablation has occurred.

The treatment parameters and observations from the first set of RF applications are shown in Table 1. The effect of the treatment was evaluated endoscopically. The areas of the esophagus treated (the "treatment patterns") were clearly visible as white bands. Untreated areas had the normal red/pink color.

**TABLE 1**

| **Treatment Set 1: Parameters and Observations** | | | |
|---|---|---|---|
| | | Observed Impedance | |
| Device Location & Configuration | Treatment Protocol | Initial (Ohms)¹ | Terminal (Ohms) |
| | | | |
| Distal//Bipolar | 25 watts @ 30 secs + | 33 | 258 |
| | 40 watts @ 30 secs | | |
| Monopolar Band 1 | 25 watts @ 30 secs | 125 | Shut off at 29 secs² |
| Band 2 | 25 watts @ 30 secs | 107 | Shut off at 20 secs |
| Band 3 | 25 watts @ 30 secs | 125 | Shut off at 25 secs |
| Band 4 | 25 watts @ 30 secs | 105 | Shut off at 22 secs |
| Band 5 | 25 watts @ 30 secs | 125 | Full³ at 30 secs |
| Band 6 | 25 watts @ 30 secs | 90 | Shut off at 19 secs |
| Proximal // Bipolar | 15 watts @ 30 secs + | No data | No change from |
| | 40 watts @ 30 secs | | baseline |

| | | | |
|---|---|---|---|
| Transformer tap = 50 Shut off usually occurs at 300 ohms. "Full" indicates treatment progressed for the entire scheduled interval without an automatic termination event. | | | |

As can be seen from the table, once the observed impedance at the ablation site reached 300 ohms the radio frequency generator shut off the signal.

The treatment parameters and observations from the second set of RF applications made mid level in the esophagus are shown in Table 2. As before the effect of the treatment was evaluated endoscopically. The treatment patterns were clearly visible.

**TABLE 2**

| **Treatment Set 2: Parameters and Observations** | | | |
|---|---|---|---|
| | | Observed Impedance | |
| Device Location & Configuration | Treatment Protocol | Initial (Ohms)⁴ | Terminal (Ohms) |
| | | | |
| Distal// Bipolar | 25 watts @ 60 sees | 30 | 121 |
| | | | (jump at 30 secs) |
| Monopolar Band 1 | 20 watts @ 60 sees | 112 | 103 |
| | | | Full at 60 secs⁵ |
| Band 2 | 20 watts @ 60 secs | 108 | 300 |
| | | | Shut off at 25 secs |
| Band 3 | 20 watts @ 60 secs | 109 | 301 |
| | | | Shut off at 31 secs |
| Band 4 | 20 watts @ 60 secs | 108 | 300 |
| | | | Shut off at 27 secs |
| Band 5 | 20 watts @ 60 secs | 115 | 301 |
| | | | Shut off at 42 secs |
| Band 6 | 20 watts @ 60 secs | 109 | 301 |
| | | | Shut off at 24 secs |
| Proximal // Bipolar | 40 watts @ 60 secs | 32 | 37 |

| | | | |
|---|---|---|---|
| Transformer tap = 50 "Full" indicates treatment progressed for the entire scheduled interval without an automatic termination event. | | | |

The treatment parameters and observations from the third set of RF applications are depicted in Table 3. The effect of the treatment was evaluated endoscopically. The treatment patterns were clearly visible as white bands as compared to the normal red/pink color.

**TABLE 3**

| **Treatment Set 3: Parameters and Observations** | | | |
|---|---|---|---|
| | | Observed Impedance | |
| Device Location & Configuration | Treatment Protocol | Initial (Ohms)⁶ | Terminal (Ohms) |
| | | | |
| Distal// Bipolar | 25 watts @ 120 secs | 67 | 168 |
| | | | Dec at 106 secs |
| ⁷Monopolar Band | 15 watts @ 90 secs | 104 | 283 |
| 1 | | | Full at 90 secs⁸ |
| Band 2 | 15 watts @ 90 secs | 110 | 301 |
| | | | Shut off at 37 secs |
| Band 3 | 15 watts @ 90 secs | 115 | 300 |
| | | | Shut off at 43 secs |
| Band 4 | 15 watts @ 90 secs | 105 | 287 |
| | | | Full at 90 secs |
| Band 5 | 15 watts @ 90 secs | 104 | 281 |
| | | | Full at 90 secs |
| Band 6 | 15 watts @ 90 secs | 105 | 289 |
| | | | (inc at 38 secs) |
| Proximal // Bipolar | 40 watts @ 120 secs | 87 | 105 |

| | | | |
|---|---|---|---|
| Bipolar transformer tap = 35; Monopolar = 50 Monopolar treatment usually resulted in a dramatic decrease in "watts" read out within the middle and the end of the treatment interval. The decrease was from 15 watts (initial setting) to 3 or 4 watts at the end of the treatment cycle. "Full" indicates treatment progressed for the entire scheduled interval without an automatic termination event. | | | |

The treatment transformer tap was changed for the bipolar treatments from 50 to 35. Of note is the observation that towards the end of the monopolar treatments, the watts output as reported on the generator decreased from a setting of 15 watts to a reading of 3 to 4 watts. The increase in impedance observed in the study may be useful as an endpoint for controlling the RF energy at the ablation site.

The RF energy can be applied to the electroconductive members in a variety of ways. In one embodiment, it is applied in the bipolar mode to the bipolar rings through simultaneous activation of alternating rings. In another embodiment, it is applied to the bipolar rings through sequential activation of pairs of rings. In another embodiment, the RF energy can be applied in monopolar mode through sequential activation of individual monopolar bands or simultaneous activation of the monopolar bands.

After the treatment of the swine esophagus as described above using radio frequency, the esophagus was extirpated and fixed in 10 percent normal buffered formalin (NBF). Three distinct lesion areas were observed corresponding to the three treatment sites and the esophagus was divided into three sections that approximated the three treatment zones. Each segment was cut into 4 to 5 mm thick serial cross sections. Selected sections from each treatment segment were photographed and the photographs of representative treatment segments were assembled side by side to compare similar catheter electrode patterns among the three treatment regimens. The following observations were made. Almost all the treated segments demonstrated necrosis of the mucosa. Changes with the submucosal, muscularis and adventitial layers were observed, typically demonstrated by tissue discoloration suggestive of hemorrhage within the tissue. Finally in comparing the tissue to the normal esophageal morphology, most treated segments were dilated with thinned walls. Thus, all the electrode patterns and treatment parameters resulted in ablation of the mucosal layer of the esophagus.

The treated esophagus was sectioned into 44 sections with each section labeled as either a treatment region or a region adjacent to a treatment region. Each section was processed for histological examination and stained with H&E and reviewed twice. The following parameters were estimated and noted.
a. Percent Epithelial Slough:
   Slough was defined as a separation of one or more layers of the epithelium as visualized at 100-x magnification.
b. Epith: Percent cell death:
   The basal layers of the epithelium were reviewed at 400-x magnification. Determination of "cell death" was based upon the following criteria:
      Condensation of the nuclear material.
      Loss of well-defined nuclear outline.
      Loss of well-defined cellular detail.
c. Lamina propria// Muscularis mucosa// Submucosa:
   Percent death:
      Cell death was based primarily on the condensation of nuclear material.
d. Muscularis/Adventitia:
   Same as above.

The following table summarizes the percent slough, percent death in the mucosa and submucosa and percent death in the muscularis as determined during the above-described study.

**TABLE 4**

| Section Number | Section Location | | Percent Slough | Percent death //. Mucosa & submucosa | Percent death // Muscularis |
|---|---|---|---|---|---|
| 1 | Distal spacer | | 0 | 0 | 0 |
| 2 | Distal // Bipolar Ring | | 0 | 0 | 0 |
| 3 | Distal // Bipolar Ring | | 33 | 100 | 75 |
| 4 | Distal // Bipolar Ring | | 100 | 100 | 50 |
| 5 | Distal // Monopolar Band | | 100 | 100 | 75 |
| 6 | Distal // Monopolar Band | | 100 | 100 | 75 |
| 7 | Distal // Null band | | 100 | 100 | 50 |
| 8 | Distal // Null band | | 100 | 100 | 75 |
| 9 | Distal // Bipolar axial | | 50 | 95 | 50 |
| 10 | Distal // Bipolar axial | | 75 | 90 | 25 |
| 11 | Distal // Bipolar axial | | 50 | 75 | 25 |
| 12 | Distal // Bipolar axial | | 50 | 75 | 25 |
| 13 | Distal // Bipolar axial | | 50 | 100 | 25 |
| 14 | Distal ◇ Mid spacer | | 0 | 0 | 0 |
| 15 | Distal ◇ Mid spacer | | 0 | 0 | 0 |
| 16 | Distal ◇ Mid spacer | | 0 | 0 | 0 |
| 17 | Distal ◇ Mid spacer | | 0 | 0 | 0 |
| 18 | Distal ◇ Mid spacer | | 5 | 5 | 5 |
| 19 | Mid tmt // Bipolar ring | | 75 | 100 | 25 |
| 20 | Mid tmt // Bipolar ring | | 60 | 100 | 25 |
| 21 | Mid tmt // Bipolar ring | | 90 | 100 | 25 |
| 22 | Mid tmt // Monopolar band | | 60 | 75 | 25 |
| 23 | Mid tmt // Null band | | 65 | 95 | 10 |
| 24 | Mid tmt // Null band | | 75 | 100 | 10 |
| 25 | Mid tmt // Bipolar axial | | 65 | 95 | 10 |
| 26 | Mid tmt // Bipolar axial | | 35 | 25 | 25 |
| 27 | Mid tmt // Bipolar axial | | 25 | 25 | 10 |
| 28 | Mid tmt // Bipolar axial | | 30 | 50 | 25 |
| 29 | Mid tmt ◇ proximal spacer | | 65 | 25 | 50 |
| 30 | Proximal // Bipolar ring | | 50 | 75 | 50 |
| 31 | Proximal // Bipolar ring | | 25 | 75 | 25 |
| 32 | Proximal // Bipolar ring | | 50 | 80 | 25 |
| 33 | Proximal // Bipolar ring | | 75 | 75 | 50 |
| 34 | Proximal // Monopolar band | | 90 | 50 | 50 |
| 35 | Proximal // Monopolar band | | 100 | 99 | 75 |
| 36 | Proximal // Monopolar band | | 100 | 100 | 75 |
| 37 | Proximal // Null band | | 90 | 95, | 75 |
| 38 | Proximal // Bipolar axial | | 50 | 25 | 50 |
| 39 | Proximal // Bipolar axial | | 90 | 50 | 50 |
| 40 | Proximal // Bipolar axial | | 100 | 75 | 75 |
| 41 | Proximal // Bipolar axial | | 90 | 90 | 50 |
| 42 | Proximal spacer | | 0 | 0 | 0 |
| 43 | Proximal spacer | | 0 | 0 | 0 |
| 44 | Proximal spacer | | 0 | 0 | 0 |

A particularly preferred embodiment of a device of the invention is shown in Figure 8. This device comprises an esophageal electrode balloon catheter 110 comprised of two electrode arrays, 112 and 114, respectively, affixed to the outside of an 18.25mm diameter x 40mm long balloon 130 that is mounted on a 3 foot long catheter 160. One electrode 112 is aligned with the edge 116 that intersects the taper region located at the distal end of the balloon 132 while the other 114 is aligned with the proximal intersecting taper edge located at the proximal end of the balloon 134.

Figure 9 shows a bottom view 150 and a top view 152 of the electrode arrays. Each array comprises 20 parallel bars, 0.25mm wide x 60-65mm long, separated by gaps of 0.3mm. When adhered to the balloon, the bars on the circuit form 20 complete continuous rings around the circumference. The electrode array itself is etched from a laminate consisting of copper on both sides of a polyimide substrate. One end of each copper bar has a small plated through-hole 154, which allows signals to be passed to these bars from 1 of 2 copper junction blocks 156 and 158, respectively, on the back of the laminate. One junction block 156 is connected to all of the even numbered bars, while the other 158 is connected to all of the odd numbered bars.

As shown in Figure 8, each junction block is then wired to a bundle of 5 34 AWG wires 136. The wiring is external to the balloon, with the distal circuit wires affixed beneath the proximal circuit. Upon meeting the shaft of the catheter, these 4 bundles are then soldered to 3 litz wire bundles. One bundle serves as a common conductor for both circuits while the other 2 bundles are wired individually to each of the two circuits. The litz wires are encompassed with heat-shrink tubing along the entire length of the shaft of the catheter. Upon emerging from the proximal end of the catheter, each of these bundles 138 is individually insulated with heat-shrink tubing before terminating to a mini connector plug 140.

The y-connector 142 at the proximal end of the catheter includes access ports for both the thru lumen 144 and the inflation lumen 146. The thru lumen spans the entire length of the balloon catheter and terminates with a flexible lumen tip 148 at the distal end of the balloon.

For delivery of the device, the balloon is folded and placed within a sheath (not shown). During deployment, this sheath is retracted along the shaft to expose the electrode balloon.

The device shown in Figure 8 is designed for use with the radiofrequency energy method described herein. Preferably, the electrode arrays are activated with approximately 40 watts of radio frequency power for the length of time necessary to deliver from 200 to 600 joules of energy to the tissue. Since the total treatment area of a 1 centimeter long electrode array wrapped around an 18.25 millimeter diameter balloon is about 5.7 square centimeters, this equates to approximately 35 to 105 joules per square centimeter of esophageal area. For a device employing a different length electrode array or a different diameter balloon, the desired power and energy settings would be scaled as needed to deliver the same power and energy per unit area. These changes could be made either automatically or from user input to the radio frequency power source. If different treatment depths are desired, electrode geometry can be modified to create either a deeper or more superficial treatment region. Making the bipolar electrode rings more narrow and spacing them closer together will reduce the treatment depth. Making the bipolar electrode rings wider and spacing them further apart will increase the depth of the treatment region. Non-uniform widths and spacings may be exploited to achieve various treatment effects. As described, in a method of the invention using a device of the invention wherein radiofrequency energy is applied to the tissue to be ablated, the depth of ablation may be controlled by proper selection of the treatment settings. For the device of Figure 8, wherein the electrode array having a length of about 1 centimeter long and a diameter of about 18 mm, it is desirable to provide power in the range of 20-60 watts for a time period between 5 and 20 seconds.

In order to ensure good contact between the esophageal wall and the electrode array, slight suction may be applied to the thru-lumen tube to reduce the air pressure in the esophagus distal to the balloon, and slight suction could simultaneously be applied to the portion of the esophagus proximal to the balloon. This suction would cause the portion of the esophageal wall distended by the balloon to be pulled against the electrode array located on the balloon.

Various modifications to the above-mentioned treatment parameters with the electrode arrays can be made to optimize the ablation of the abnormal tissue. To obtain shallower lesions than the ones obtained in the above-mentioned study the RF energy applied may be increased while decreasing the treatment time. Also, the electrode patterns may be modified such as shown in Figure 7 to improve the evenness and shallowness of the resulting lesions. The system and method of the invention may also be modified to incorporate temperature feedback, resistance feedback and/or multiplexing electrode channels.

While a preferred embodiment of the present invention has been described, it should be understood that various changes, adaptations and modifications may be made therein.

## Claims

1. A device for ablating abnormal mucosal tissue from a human esophagus, comprising:
(a) a balloon catheter comprising an inflatable balloon (130) positioned on the distal end of a catheter (160);
(b) energy distribution means comprising two individually wired electrode arrays (112, 114) positioned around a circumference of a predetermined length of the balloon capable of distributing radio frequency energy, each electrode array comprising bipolar rings; and
(c) power means configured to power the energy distribution means so that energy is applied to the tissue at levels appropriate to ablate the tissue to a predetermined depth of ablation.

2. The device of claim 1, wherein the means is adapted to
activate the electrode arrays (112, 114) with approximately 40 watts of radio frequency power for a length of time necessary to deliver from 200 to 600 joules of energy to the tissue.

3. The device of claim 1 or claim 2, wherein the power means is adapted to
activate the electrode arrays (112, 114) with approximately 40 watts of radio frequency power for a length of time necessary to deliver from approximately 35 to 105 joules per square centimeter of esophageal area.

4. The device of any preceding claim, wherein each electrode array (112, 114) is etched from a laminate consisting of copper on both sides of a polyimide substrate.

5. The device of any preceding claim, wherein the device includes a vacuum or suction element for pulling the esophageal wall against the outside circumference of the balloon (130).

6. The device of any preceding claim, wherein the depth of ablation is controllable by selection of treatment settings.

7. The device of any preceding claim, wherein the power means is adapted to allow the user to select an appropriate treatment time and power setting to obtain a controlled depth of ablation.

8. The device of any preceding claim, wherein the power means is configured to provide a predetermined amount of energy to the energy distribution means for a predetermined period of time sufficient to provide the energy distribution means with sufficient power to apply the appropriate level of energy to the tissue to achieve an ablation depth that does not extend beyond the submucosa layer of the esophagus.

9. The device of claim 8, wherein the power means further comprises a controller.

10. The device of claim 9, wherein the controller comprises a computer and software.

11. The device of any preceding claim, wherein the bipolar rings are spaced one from another by no more than 2mm.

## Patentansprüche

1. Vorrichtung zum Abladieren von abnormalem Schleimhautgewebe aus einer menschlichen Speiseröhre, wobei die Vorrichtung folgendes umfaßt:
(a) einen Ballonkatheter, welcher einen aufblasbaren Ballon (130) umfaßt, der am distalen Ende eines Katheters (160) positioniert ist,
(b) Energieverteilungsmittel, umfassend zwei individuell verdrahtete Elektrodenarrays (112, 114), die um einen Umfang einer vorbestimmten Länge des Ballons positioniert sind und in der Lage sind, Hochfrequenzenergie zu verteilen, wobei jedes Elektrodenarray bipolare Ringe umfaßt, und
(c) Energieversorgungsmittel, die dafür ausgestaltet sind, die Energieverteilungsmittel so mit Energie zu versorgen, daß Energie in Mengen auf das Gewebe angewendet wird, die ausreichend sind, um das Gewebe bis zu einer vorbestimmten Ablationstiefe zu abladieren.

2. Vorrichtung nach Anspruch 1, wobei die Mittel so ausgestaltet sind, daß sie die Elektrodenarrays (112, 114) mit ungefähr 40 Watt Hochfrequenzenergie antreiben, für eine Zeitdauer, die notwendig ist, um dem Gewebe 200 bis 600 Joule an Energie zuzuführen.

3. Vorrichtung nach Anspruch 1 oder Anspruch 2, wobei die Energieversorgungsmittel so ausgestaltet sind, daß sie die Elektrodenarrays (112, 114) mit ungefähr 40 Watt Hochfrequenzenergie aktivieren, für eine Zeitdauer, die erforderlich ist, um ungefähr 35 bis 105 Joule pro Quadratzentimeter an Speiseröhrenfläche zuzuführen.

4. Vorrichtung nach einem der vorangegangenen Ansprüche, wobei jedes Elektrodenarray (112, 114) aus einem Laminat, bestehend aus Kupfer auf beiden Seiten eines Polyimidsubstrats, geätzt ist.

5. Vorrichtung nach einem der vorangegangenen Ansprüche, wobei die Vorrichtung ein Vakuum- oder Saugelement umfaßt, um die Speiseröhrenwand gegen den äußeren Umfang des Ballons (130) zu ziehen.

6. Vorrichtung nach einem der vorangegangenen Ansprüche, wobei die Ablationstiefe durch Auswahl von Behandlungseinstellungen steuerbar ist.

7. Vorrichtung nach einem der vorangegangenen Ansprüche, wobei die Energieversorgungsmittel so ausgestaltet sind, daß sie es dem Benutzer erlauben, eine geeignete Behandlungszeit und Energieeinstellung auszuwählen, um eine kontrollierte Ablationstiefe zu erhalten.

8. Vorrichtung nach einem der vorangegangenen Ansprüche, wobei die Energieversorgungsmittel so konfiguriert sind, daß sie den Energieverteilungsmitteln eine vorbestimmte Menge an Energie bereitstellen, für eine vorbestimmte Zeitdauer, die ausreichend ist, um den Energieverteilungsmitteln ausreichend Energie bereitzustellen, um das geeignete Energieniveau auf das Gewebe anzuwenden, um eine Ablationstiefe zu erreichen, die sich nicht über die Submucosaschicht der Speiseröhre hinaus erstreckt.

9. Vorrichtung nach Anspruch 8, wobei die Energieversorgungsmittel weiterhin eine Steuerung umfassen.

10. Vorrichtung nach Anspruch 9, wobei die Steuerung einen Computer und Software umfaßt.

11. Vorrichtung nach einem der vorangegangenen Ansprüche, wobei die bipolaren Ringe um nicht mehr als 2 mm voneinander beabstandet sind.

## Revendications

1. Dispositif pour l'ablation de tissu muqueux anormal d'un oesophage humain, comportant :
(a) un cathéter à ballonnet comportant un ballonnet gonflable (130) positionné sur l'extrémité distale d'un cathéter (160) ;
(b) un moyen de distribution d'énergie comportant deux rangées d'électrodes (113, 114) câblées individuellement, positionnées le long d'une circonférence d'une longueur prédéterminée du ballonnet, capables de distribuer de l'énergie radiofréquence, chaque rangée d'électrodes comportant des anneaux bipolaires ; et
(c) un moyen d'alimentation configuré pour alimenter le moyen de distribution d'énergie afin que de l'énergie soit appliquée au tissu à des niveaux appropriés pour réaliser une ablation du tissu jusqu'à une profondeur prédéterminée d'ablation.

2. Dispositif selon la revendication 1, dans lequel le moyen est conçu pour
activer les rangées d'électrodes (112, 114) avec approximativement 40 watts de puissance radiofréquence pendant un intervalle de temps nécessaire pour délivrer 200 à 600 joules d'énergie au tissu.

3. Dispositif selon la revendication 1 ou la revendication 2, dans lequel le moyen d'alimentation est conçu pour
activer les rangées d'électrodes (112, 114) avec approximativement 40 watts de puissance radiofréquence pendant un intervalle de temps nécessaire pour délivrer approximativement 35 à 105 joules par centimètre carré de surface oesophagienne.

4. Dispositif selon l'une quelconque des revendications précédentes, dans lequel chaque rangée d'électrodes (112, 114) est gravée dans un stratifié comprenant du cuivre sur les deux faces d'un substrat de polyimide.

5. Dispositif selon l'une quelconque des revendications précédentes, lequel dispositif comprend un élément à dépression ou d'aspiration destiné à tirer la paroi oesophagienne contre la circonférence extérieure du ballonnet (130).

6. Dispositif selon l'une quelconque des revendications précédentes, dans lequel la profondeur d'ablation peut être commandée par un choix de réglages de traitement.

7. Dispositif selon l'une quelconque des revendications précédentes, dans lequel le moyen d'alimentation est conçu pour permettre à l'utilisateur de choisir un temps de traitement et un réglage de puissance appropriés pour obtenir une profondeur d'ablation commandée.

8. Dispositif selon l'une quelconque des revendications précédentes, dans lequel le moyen d'alimentation est configuré pour fournir une quantité prédéterminée d'énergie au moyen de distribution d'énergie pendant une période de temps prédéterminée suffisante pour fournir au moyen de distribution d'énergie une puissance suffisante pour appliquer le niveau approprié d'énergie au tissu afin d'obtenir une profondeur d'ablation n'allant pas au-delà de la couche sous-muqueuse de l'oesophage.

9. Dispositif selon la revendication 8, dans lequel le moyen d'alimentation comporte en outre une unité de commande.

10. Dispositif selon la revendication 9, dans lequel l'unité de commande comprend un ordinateur et un logiciel.

11. Dispositif selon l'une quelconque des revendications précédentes, dans lequel les anneaux bipolaires sont espacés l'un de l'autre de pas plus de 2 mm.
